# EUROPEAN PATENT APPLICATION

(11) **EP 1 133 987 A1**
(43) Date of publication of application: **19.09.2001**
(21) Application number: 00301951.0
(22) Date of filing: 09.03.2000
(51) Int. Cl.: A61K 31/70, A61K 45/06

(54) **Treatment of inflammatory dermatoses with combinations of erythromycin or clarythromycin, metronidazole and a hydrogen pump inhibitor**

(71) Applicant: Whitcroft, Ian, Huron, SD 57350 (US); Diaz, Camilo, Birmingham B9 4PQ (GB)
(72) Inventor: Whitcroft, Ian, Huron, SD 57350 (US); Diaz, Camilo, Birmingham B9 4PQ (GB)
(74) Representative: Hucker, Charlotte Jane

(57) **Abstract**

Use of i) erythromycin, clarythromycin or an analogue or derivative thereof; ii) metronidazole or an analogue or derivative thereof; and iii) omeprazole or an analogue or derivative thereof, for the manufacture of a medicament for the treatment of a condition selected from perioral papular or pustular acne, and similar inflammatory papulopustular dermatoses and perioral dermatitis.

## Description

### Field of the Invention

The present invention relates to the treatment of skin conditions such as acne rosacea, pustular or papular acne, and similar inflammatory pustular or papular dermatoses.

### Background to the Invention

Rosacea is a chronic inflammatory disease which affects the face. It is characterised by episodic flushing, erythema, and telangiectasia affecting the cheeks, chin, forehead, and nose. It may be further complicated by inflammatory swelling, papules and pustules. It is observed more frequently in women than in men, with an age of onset between 30 and 50, most commonly in those of Celtic or northern European descent.

Current treatments of rosacea include long-term the use of systemic or topical antibiotics, such as tetracycline, minocycline, doxicycline, erythromycin and metronidazole. However, such treatments are ineffective in achieving eradication of the condition, even for relatively short periods of time.

Many theories have been proposed to explain the aetiology of rosacea, with a view to finding alternative treatments thereof. An association with gastrointestinal disease has been considered as long ago as 1920 when achlorhdria and hypochlorohydria were thought to be predisposing factors. However, attempts to confirm such an association have been unsuccessful. Hypersensitivity to *Demodex folliculorum,* a mite which inhabits the facial pilosebaceous follicle, has also been postulated to be of relevance, and increased numbers of *Demodex* mites have been demonstrated in rosacea.

The association between rosacea and gastrointestinal disease has been revisited since the discovery that the Gram-negative bacterium *Helicobacter pylori (H.Pylori)* is the cause of gastritis and duodenal ulcer disease. Based on case-series studies of rosacea, several authors have reported higher than expected *H*. *pylori* seroprevalance and antibody titres, and have commented on improvement in the dermatological condition following *H*. *pylori* eradication therapy; see Rebora et al, Dermatology (1995) 89:1603-1604, Kolibasora et *al*, Archives of Dermatology (1996) 132:1393, and Utas et al, J.AM.Acad. Dermatology (1999) 40:433-435. Yet none of these studies employed proper controls for their results to be considered meaningful. Furthermore, a number of other studies have failed to confirm the alleged relationship between *H.Pylori* and rosacea; see Jones et al, Archives of Dermatology (1998) 134:511, and Bamford et *al,* Archives of Dermatology (1999) 135:659-663.

The situation in this respect, therefore, remains conflicting and unclear, as does the cause of this common, yet poorly understood, facial dermatosis.

### Summary of the Invention

Surprisingly, and as a result of case-controlled studies, we have now found that distinct types of rosacea, and in particular acne rosacea, and similar inflammatory skin dermatoses, can be effectively treated by administering to a patient a treatment regime comprising of i) erythromycin, clarythromycin or an analogue or derivative thereof, ii) metronidazole or an analogue or derivative thereof, and iii) a gastrointestinal hydrogen pump inhibitor such as omeprazole, or an analogue or derivative thereof.

Prior to the present invention, and for some time now, it has been believed that the different signs of rosacea are merely symptomatic of different, progressive, stages of the same general condition. Specifically, it has been suggested that the seemingly lesser signs of inflammatory rosacea (erythema and telangiectasis) progress to lesions and/or papulopustular signs (pimples on the skin). We believe that this may not be true, as patients have been observed to exhibit either inflammatory signs or papulopustular signs. Instead, the different signs may represent distinct types of rosacea.

At present, it is not understood how the triple drug combination according to the present invention functions in achieving the desired results. One possibility is that there may be a causal relationship between the dermatoses to be treated and *H*.*pylori*, such that eradication of *H.pylori* may lead to resolution of these dermatoses. Another possibility is that as, in certain instances, the presence of *H*.*pylori* has been found in pustules on the skin, the triple drug combination may act directly to eradicate that external form of *H*.*pylori*. Yet another possibility is that the triple drug combination works in another manner, such that any apparent relationship between its use in the present invention and *H*.*pylori* may simply be coincidental, such that further study will be necessary to elucidate its precise mode of action.

### Description of the Invention

The preferred treatment regime comprises, in combination, erythromycin or clarythromycin, metronidazole and omeprazole, and the most preferred treatment regime comprises clarythromycin, metronidazole and omeprazole. As mentioned above, however analogues or derivatives of any of these drugs may be used provided that their combined administration achieves the desired result. For instance, another similar-acting macrolide antibiotic to erythromycin or clarithromycin may be used. Similarly, lanzoprazole, or any other drug which acts to inhibits a gastrointestinal hydrogen pump, may be used instead of omeprazole. Additionally, other drugs may be useful which have a similar or equivalent pharmacological mode of action to the drugs named above.

Treatment may be by administration of the three drugs by any suitable means. Conveniently, however, the three drugs will be administered orally, by topical application to a patient's skin, or parenterally.

If the combination is to be administered orally, it may take the form of separate pills containing each of the separate drugs, for simultaneous or sequential administration. Preferably, however, the three drugs will be combined into a single dosage form, preferably a tablet or capsule, for patient convenience. In the case of topical or parenteral administration, the drugs are incorporated into a suitable pharmaceutically-acceptable carrier. Such formulations have not been disclosed in the prior art, and constitute further aspects of the claimed invention.

The relative proportions of the three drugs necessary for effective treatment may vary depending on the route of administration, and the particular patient to be treated. Typically, however, a patient will receive 200 to 600 mg, preferably 300 to 500 mg of erythromycin, clarythromycin or an analogue thereof, 200 to 500 mg, preferably 300 to 500 mg, metronidazole, and 10 to 100 mg, preferably 20 to 60 mg, omeprazole, on a daily basis. A particularly preferred treatment regime, however 250 mg erythromycin, clarythromycin or an analogue thereof, about 400 mg metronidazole and about 20 mg omeprazole. Treatment periods vary from patient to patient, from one week to a number of years.

The results upon which the present invention are based are summarised in the following example.

### Example

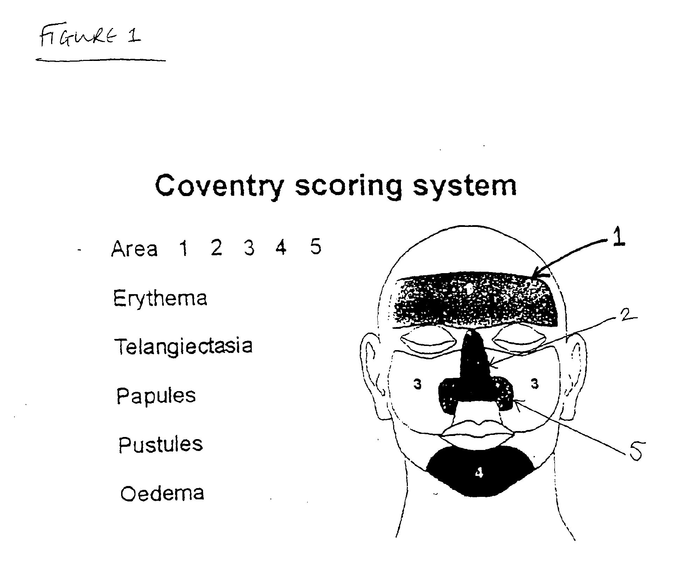

42 patients with rosacea and evidence of infection with *H*.*pylori* were studied. At week 0, an initial assessment of the severity of each of the patient's skin disease was made, using the so-called "Coventry Scoring System". This scoring system, developed in the Dermatology Department at Walsgrave Hospital, Coventry, UK, measures the severity of rosacea in five different facial areas and for five different parameters. The different facial areas are depicted in Figure 1, below. The different parameters are erythema, telangiectasia, papules, pustules and oedema. A score of one is given if less than the area involved is affected, and a score of two if half or more of the area is affected.

Each patient was subjected to clinical photography, and then treated with an oral dosage of 20 mg omeprazole, 400 mg metronidazole, and 250 mg clarythromycin for one week. Disease severity scoring and clinical photography were repeated at the end of weeks 6, 12, 18, and 24, and fourteen patients were followed for a period of 52 weeks.

At week 0 the mean severity score was 10 (range 3-24). Following treatment, at week 6 the mean score had fallen to 5.8 (range 2-12), falling further to 4.6 (range 2-8) at week 12. At weeks 18 and 24 the mean severity scores were 4.5 (range 2-12) and 5.6 (range 2-14) respectively. There was a significant difference between scores at week 0 and weeks 6 and 12 (p<0.0001), and also between scores at week 0 and weeks 18, and 24 (p<0.001). Five relapses were recorded, one at week 18 and four at week 24, with rosacea severity scores returning to values seen at week 0.

The study demonstrates that the combined treatment of omeprazole, metronidazole and clarythromycin was effective in the treatment of symptoms associated with rosacea in general. It was noted in particular that the treatment significantly reduced red papules and/or white pustules on patients' faces.

While the study treated patients with evidence of infection with *H.pylori,* because a causal relationship between successful treatment of acne rosacea and eradication of *H.pylori* is yet to be confirmed, it is believed that the triple drug combination of the present invention may be useful in the treatment of patients who are not infected with *H*.*pylori*.

## Claims

1. Use of
i) erythromycin, clarythromycin or an analogue or derivative thereof;
ii) metronidazole; or an analogue or derivative thereof; and
iii) a gastrointestinal hydrogen pump inhibitor,
for the manufacture of a medicament for the treatment of a condition selected from papulopustular rosacea and similar inflammatory papulopustular dermatoses.

2. Use according to claim 1, wherein the condition to be treated is perioral papular or pustular acne.

3. Use according to claim 1 or claim 2, wherein the gastrointestinal hydrogen pump inhibitor is omeprazole or an analogue or derivative thereof.

4. Use according to any preceding claim, wherein the medicament is for oral administration.

5. Use according to any of claims 1 to 3, wherein the medicament is for topical administration.

6. Use according to any of claims 1 to 3, wherein the medicament is for parenteral administration.

7. A dosage form comprising, in a single unit, a combination of
i) erythromycin, clarythromycin or an analogue or derivative thereof;
ii) metronidazole or an analogue or derivative thereof; and
iii) a gastrointestinal hydrogen pump inhibitor.

8. A dosage form according to claim 7, wherein the gastrointestinal hydrogen pump inhibitor is omeprazole or an analogue or derivative thereof.

9. A dosage form according to claim 7 or claim 8, which is a tablet or capsule.

10. A topical pharmaceutical composition comprising, in a pharmaceutically-acceptable carrier, a combination of
i) erythromycin, clarythromycin or an analogue or derivative thereof;
ii) metronidazole or an analogue or derivative thereof; and
iii) a gastrointestinal hydrogen pump inhibitor, such as omeprazole or an analogue or derivative thereof.
